# EUROPEAN PATENT APPLICATION

(11) **EP 2 065 033 A1**
(43) Date of publication of application: **03.06.2009**
(21) Application number: 09151789.6
(22) Date of filing: 10.03.2006
(51) Int. Cl.: A61K 9/107, A61K 9/48, A61K 31/12, A61K 47/14, A61K 47/26, A61K 47/44

(54) **Microemulsions of cannabinoid receptor binding compounds**

(30) Priority: 10.03.2005 GB 0504950
(62) Divisional of application: 06723358.5
(71) Applicant: Novartis AG, 4056 Basel (CH)
(72) Inventor: Galli, Bruno, 4411, Seltisberg (CH); Rigassi, Thomas, 4106, Therwil (CH)
(74) Representative: Vögeli-Lange, Regina

(57) **Abstract**

The present invention relates to a novel spontaneously dispersible pharmaceutical composition, e. g. a microemulsion preconcentrate, in which the active drug substance is a cannabinoid receptor binding compound, in particular a corresponding naphthalene derivative, that is useful, e. g., for the treatment or prevention of chronic pain.

## Description

The present invention relates to novel pharmaceutical compositions, in which the active drug substance is a cannabinoid receptor binding compound, in particular a corresponding naphthalene derivative, that is useful, e. g., for the treatment or prevention of chronic pain, especially inflammatory, e. g. chronic inflammatory, pain, inflammatory diseases, for example inflammatory airways diseases, e. g. COPD or asthma, rhinitis, inflammatory bowel disease, cystitis, e. g. interstitial cystistis, pancreatitis, uveitis, hyperalgesia, allergic responses, pain and/or inflammation and/or oedema consequential to trauma, e. g. associated with burns, sprains, fractures or the like, or subsequent to surgical intervention, e. g. associated with post-operative conditions, inflammatory pain of diverse genesis, e. g. bone and joint pain, e. g. osteoarthritis, rheumatoid arthritis, rheumatic disease, teno-synovitis, gout, cancer pain, myofascial pain, e. g. muscular injury or fibromyalgia, neuropathic, e. g. chronic neuropathic, pain, e. g. diabetic neuropathy, post-herpetic neuralgia or phantom limb pain, perioperative pain, e. g. associated with general surgery or gynecologic surgery, pain associated with, e. g., angina or menstruation, inflammatory skin disorders, for example psoriasis or eczema, spasms of the gastro-intestinal tract or uterus, glaucoma, irregular intraocular pressure, Crohn's disease, ulcerative colitis, and muscle spasticity and tremor in, e. g., multiple sclerosis.

Cannabinoid receptor binding naphthalene derivatives are a class of compounds described, e. g., in WO-2002/42248, the contents of which publication are herewith incorporated herein by reference.

Cannabinoid receptor binding naphthalene derivatives, such as those disclosed in WO-2002/42248, present highly specific difficulties in relation to administration generally and to galenic compositions in particular, including in particular problems of drug bioavailability and variability in inter- and intra-subject, e. g. -patient, dose response, necessitating the development of a non-conventional dosage form.

It has now surprisingly been found, that stable pharmaceutical compositions of cannabinoid receptor binding naphthalene derivatives with particularly interesting bioavailability characteristics and reduced variability in inter- and intra-subject bioavailability parameters, are obtainable. These novel compositions may overcome or substantially reduce the difficulties encountered previously. The compositions of the invention may, for example, enable the effective dosaging with concomitant enhancement of the bioavailability as well as reduced variability of the absorption/bioavailability levels for and between individual subjects. Thus, the compositions of the invention may achieve an effective therapy with tolerable dosage levels of such cannabinoid receptor binding naphthalene derivatives and may permit a closer standardization and optimization of daily dosage requirements for each individual subject. By such standardization and optimization, potential undesirable side-effects may be diminished, and the overall cost of therapy may be reduced.

Certain terms as used herein have the following meanings:

"Active agent" means the active drug substance, which is a cannabinoid receptor binding naphthalene derivative, such as those disclosed in WO-2002/42248.

Being "bioavailable" with reference to a composition of the invention means the composition provides a maximum concentration of the active agent in that composition in a use environment that is at least 1.5 fold that of a control comprising an equivalent quantity of the undispersed drug.

Unless the context requires otherwise, the word "comprise", or variations such as "comprises" or "comprising", will be understood herein to imply the inclusion of a stated integer or step or group of integers or steps, but not the exclusion of any other integer or step or group of integers or steps.

"Microemulsion" means a translucent, opalescent, slightly opaque, substantially non-opaque, or non-opaque, colloidal dispersion, that is formed spontaneously or substantially spontaneously when its components, e. g. in the form of a composition of the invention, for example in the form of a microemulsion preconcentrate, are brought into contact with an aqueous medium, for example water, e. g. by simple shaking by hand for a short period of time, for example 10 seconds, or into contact with, e. g., the gastric juices of a subject, e. g. a patient, e. g. after oral administration. A microemulsion typically comprises as colloidal structures dispersed droplets, or liquid nanoparticles, of a mean diameter of less than about 200 nm (2'000 A), generally of less than about 150 nm (1'500 A), typically of less than about 100 nm (1'000 A), generally of more than about 10 nm (100 A), as measured by standard light scattering techniques, e. g. using a Malvern ZETASIZER^{®} 3000 particle characterising machine. Solid drug particles, either crystalline or amorphous, of a mean diameter of more than about 200 nm (2'000 A) may also be present. The proportion of the particles present may be temperature dependent. A microemulsion is thermodynamically stable, e. g., for at least 15 minutes, or for up to 4 hours, or for up to 24 hours, or for more than 24 hours. A microemulsion offers, compared with a coarse emulsion, a greater ease of preparation due to its spontaneous formation, thermodynamic stability, a transparent and elegant appearance, an increased drug loading, an enhanced penetration through biological membranes, an increased bioavailability, and less inter- and intra-subject variability in drug pharmacokinetics. Further characteristics of a microemulsion can be found in: the patent GB-2,222,770; Rosof, Progress in Surface and Membrane Science, 12, 405 et seq., Academic Press (1975); Friberg, Dispersion Science and Technology, 6 (3), 317 et seq. (1985); and Muller et al., Pharm. Ind., 50 (3), 370 et seq. (1988).

"Microemulsion preconcentrate" means a composition, which spontaneously or substantially spontaneously forms a microemulsion in an aqueous medium, for example in water, for example on dilution in a composition : water ratio (weight/weight) of from 1 : 1 to 1 : 300, preferably from 1 : 1 to 1 : 70, e. g. from 1 : 5 to 1 : 70, especially from 1 : 5 to 1 : 15, e. g. of 1 : 10, or in the gastric juices of a subject, e. g. a patient, e. g. after oral administration.

Being "poorly water soluble" means having a solubility in water at 20°C of less than 1%, for example less than 0.1 %, e. g. less than 0.01% (weight/volume), i. e. being "sparingly soluble to very slightly soluble" as described in Remington, The Science and Practice of Pharmacy, 19th edition, ed. A. R. Gennaro, Mack Publishing Company, US, 1995, vol. 1, page 195.

"Spontaneously dispersible pharmaceutical composition" means a composition, preferably a microemulsion preconcentrate, that contains an active agent herein defined and is capable of spontaneously or substantially spontaneously producing colloidal structures, e. g. forming a microemulsion, when diluted with an aqueous medium, for example water, for example on dilution in a composition : water ratio (weight/weight) of from 1 : 1 to 1 : 300, preferably from 1 : 1 to 1 : 70, e. g. from 1 : 5 to 1 : 70, especially from 1 : 5 to 1 : 15, e. g. of 1 : 10, or in the gastric juices of a subject, e. g. a patient. The colloidal structures can be solid or preferably liquid particles including droplets and nanoparticles.

In a first aspect, the present invention relates to a spontaneously dispersible pharmaceutical composition comprising a cannabinoid receptor binding naphthalene derivative. This spontaneously dispersible pharmaceutical composition is herein also referred to as a "composition of the invention". A composition of the invention is preferably suitable for oral administration. An active agent in a composition of the invention is poorly water soluble as herein defined.

In a second aspect, the present invention relates to a spontaneously dispersible pharmaceutical composition comprising a cannabinoid receptor binding naphthalene derivative as an active agent and a carrier medium comprising a lipophilic component, a surfactant, and optionally a hydrophilic component. Such a composition is preferably suitable for oral administration.

In a third aspect, the present invention relates to a microemulsion preconcentrate comprising a cannabinoid receptor binding naphthalene derivative. This microemulsion preconcentrate preferably forms an oil-in-water (o/w) microemulsion when diluted with an aqueous medium, for example water, or in gastric juices. Such a microemulsion preconcentrate is preferably suitable for oral administration.

In a fourth aspect, the present invention relates to a microemulsion preconcentrate comprising a cannabinoid receptor binding naphthalene derivative as an active agent and a carrier medium comprising a lipophilic component, a surfactant, and optionally a hydrophilic component. Such a microemulsion preconcentrate is preferably suitable for oral administration. Preferably the relative proportions of the lipophilic component, the optional hydrophilic component and the surfactant lie within the "microemulsion region" on a standard three way plot graph. These phase diagrams can be generated in a conventional manner as described in e. g. GB-2,222,770 or WO-1996/13273.

In a fifth aspect, the present invention relates to a microemulsion comprising a cannabinoid receptor binding naphthalene derivative. This microemulsion is preferably an o/w microemulsion. In a preferred embodiment of the present invention, such a microemulsion is suitable for oral administration.

In a sixth aspect, the present invention relates to a microemulsion comprising a cannabinoid receptor binding naphthalene derivative as an active agent, a lipophilic component, a surfactant, water and optionally a hydrophilic component. In a preferred embodiment of the present invention, such a microemulsion is suitable for oral administration.

A preferred active agent, which is described in WO-2002/42248, includes a compound of the formula 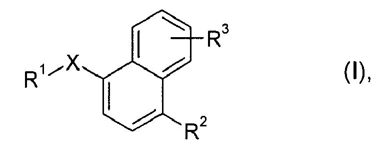 wherein
- X: is -S-, -S(=O)-, -S(=O)₂-, -S(=O)₂N(H)-, -P(=O)(OCH₃)-, -P(=O)(OH)-, -N(H)-, -N(CH₃)-, -N(H)C(=O)N(H)-, -C(=O)-, -C(=O)O-, -N(H)C(=O)-, -CH(OH)-, -CH=N-, -CH=CH-, -CH₂N(H)- or -C(=NH)-;
- R¹: is aryl or heteroaryl;
- R²: is hydrogen, OR⁴ or N(R⁵)R⁶;
R⁴ is C₁-C₈alkyl or C₂-C₈alkenyl;
R⁵ and R⁶ independently are hydrogen, C₁-C₈alkyl or C(=O)C₁-C₈alkyl; and
- R³: is hydrogen, cyano, heteroaryl, heterocycloalkyl, C(=O)R⁷, OR⁸ or N(R⁹)R¹⁰;
R⁷ is OH, C₁-C₄alkoxy, NH₂, N(H)CH₂C(=O)OH or aryl;
R⁸ is hydrogen, C₁-C₈alkyl, C(=O)C₁-C₄alkyl or C(=O)-aryl; and
R⁹ and R¹⁰ independently are hydrogen, C₁-C₈alkyl or C₂-C₄alkenyl;
   with the proviso that when X is -C(=O)- and R² and R³ are hydrogen or R² is H and R³ is 4-methoxy, R¹ is neither 1-naphthyl nor 4-methoxy-1-naphtyl;
where appropriate, in free base form or in pharmaceutically acceptable acid addition salt form.

An especially preferred active agent, which is described in WO-2002/42248, is naphthalen-1-yl-(4-pentyloxynaphthalen-1-yl)-methanone, i. e. the compound of the formula 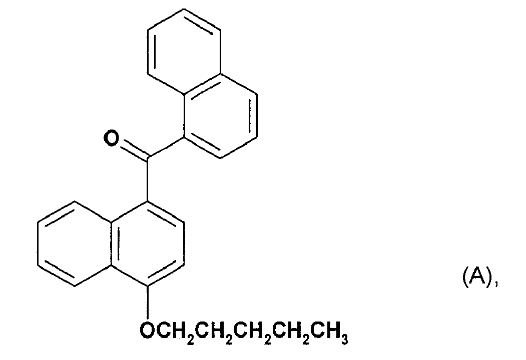 which is hereinafter referred to as "compound A".

A preferred or especially preferred active agent may be prepared as described in WO-2002/42248. As mentioned therein, the active agent may be in the form of a hydrate and/or may include other solvents, for example solvents, which may have been used for the crystallisation of a compound in solid form.

According to the present invention, an active agent may be present in a composition of the invention in an amount of up to about 20%, e. g. from about 0.05% to about 20%, preferably from about 0.5% to about 15%, more preferably from about 1.5% to about 15%, e. g. from about 1.5% to about 10%, by weight of the total composition of the invention.

Since the active agent is poorly water soluble, it is preferably carried in a carrier medium, which in one embodiment of the composition of the invention comprises a lipophilic component and a surfactant. In another embodiment, the carrier medium comprises a lipophilic component, a hydrophilic component and a surfactant.

A composition of the invention can include a variety of additives, including antimicrobial agents, antioxidants, enzyme inhibitors, flavours, preservatives, stabilizers, sweeteners and/or further additive components, such as those described in Fiedler, H. P., "Lexikon der Hilfsstoffe fur Pharmazie, Kosmetik und angrenzende Gebiete", Editio Cantor, D-7960 Aulendorf, 4th revised and expanded edition (1996). These additives may conveniently be dissolved in the carrier medium.

A composition of the invention may include a lipophilic component. The active agent may be contained in this component of the carrier medium.

A lipophilic component (when present) is preferably characterized by a low hydrophilic-lipophilic balance (HLB) value (which is preferably the mean HLB value), which is preferably lower than 10, e. g. 8 or lower.

A lipophilic component comprises one or more lipophilic substances. Suitable lipophilic substances include:

### 1) Glyceryl mono-C₆-C₁₄-fatty acid esters

These can be obtained esterifying glycerol with vegetable oil followed by molecular distillation. Monoglycerides suitable for use in a composition of the invention include both symmetric (β-monoglycerides) as well as asymmetric monoglycerides (α-monoglycerides). They also include both uniform glycerides (in which the fatty acid constituent is composed primarily of a single fatty acid) as well as mixed glycerides (in which the fatty acid constituent is composed of various fatty acids). The fatty acid constituent may include both saturated and unsaturated fatty acids having a chain length of from e. g. C₈ to C₁₄. Particularly suitable are caprylic or lauric acid monoglycerides which are commercially available, e. g. under the trade names Imwitor^{®} 308 or Imwitor^{®} 312, from, e. g., Sasol. For example, Imwitor^{®} 308 comprises at least 80% monoglycerides and exhibits the following additional characterising data: maximum free glycerol 6%, maximum acid value 3, saponification value 245-265, maximum iodine value 1, maximum water content 1%. Typically it comprises 1% free glycerol, 90% monoglycerides, 7% diglycerides, 1% triglycerides (H. Fiedler, *loc, cit.,* volume 1, page 798). A further example is Capmul^{®} MCM C8 from Abitec Corporation.

### 2) Mixtures of mono- and di-glycerides of C₆-C₁₈-fatty acids

These include both symmetric (β-monoglycerides and α,α¹-diglycerides) as well as asymmetric mono- and di-glycerides (α-monoglycerides and α,β-diglycerides) and acetylated derivatives thereof. They also include both uniform glycerides (in which the fatty acid constituent is composed primarily of a single fatty acid) as well as mixed glycerides (in which the fatty acid constituent is composed of various fatty acids) and any derivatives thereof with lactic or citric acid. The fatty acid constituent may include both saturated and unsaturated fatty acids having a chain length of from e. g. C₈ to C₁₀. Particularly suitable are mixed caprylic and capric acid mono- and di-glycerides, commercially available, e. g. under the trade names Imwitor^{®} 742 or Imwitor^{®} 928, from, e. g., Sasol. For example, Imwitor^{®} 742 comprises at least 45% monoglycerides and exhibits the following additional characterising data: maximum free glycerol 2%, maximum acid value 2, saponification value 250-280, maximum iodine value 1, maximum water 2% (H. Fiedler, *loc*. *cit*., volume 1, page 798). Other suitable mixtures comprise mono-/di-glycerides of caprylic/capric acid in glycerol, commercially available e. g. under the trade name Capmul^{®} MCM from Abitec Corporation. Capmul^{®} MCM exhibits the following characterising data: maximum acid value 2.5, minimum alpha-mono (as oleate) 80%, maximum free glycerol 2.5%, maximum iodine value 1, chain length distribution: caproic acid (C₆) maximum 3%, caprylic acid (C₈) minimum 75%, capric acid (C₁₀) minimum 10%, lauric acid (C₁₂) maximum 1.5%, maximum moisture (by Karl Fisher) 0.5%. Suitable examples of mono-/di-glcyerides with additional derivatization with lactic or citric acid are those marketed under the brand names of Imwitor^{®} 375, 377 or 380 by Sasol. Furthermore, the fatty acid constituent may include both saturated and unsaturated fatty acids having a chain length of from e. g. C₁₆ to C₁₈. A suitable example is Tegin^{®} O (glyceryl oleate) exhibiting the following characterising data: monoglyceride content 55-65%, maximum peroxide value 10, maximum water content 1%, maximum acid value 2, iodine value 70-76, saponification value 158-175, maximum free glycerol 2%.

### 3) Glyceryl di-C₆-C₁₈-fatty acid esters

These include symmetric (α,α¹-diglycerides) and asymmetric diglycerides (α,β-diglycerides) and acetylated derivatives thereof. They also include both uniform glycerides (in which the fatty acid constituent is composed primarily of a single fatty acid) as well as mixed glycerides (in which the fatty acid constituent is composed of various fatty acids) and any acetylated derivatives thereof. The fatty acid constituent can include both saturated and unsaturated fatty acids having a chain length of from C₆ to C₁₈, e. g. from C₆ toC₁₆, e. g. from C₈ to C₁₀, e. g. C₈. Particularly suitable are caprylic diglycerides, commercially available, e. g. under the trade name Sunfat^{®} GDC-S, e. g. from Taiyo Kagaku Co., Ltd.. Sunfat^{®} GDC-S has an acid value of about 0.3, a diglyceride content of about 78.8% and a monoester content of about 8.9%.

### 4) Medium chain fatty acid triglycerides

These include triglycerides of saturated fatty acids having from 6 to 12, e. g. from 8 to 10, carbon atoms. Suitable medium chain fatty acid triglycerides are those commercially available under the trade names Acomed^{®}, Myritol^{®}, Captex^{®}, Neobee^{®} M 5 F, Miglyol^{®} 810, Miglyol^{®} 812, Miglyol^{®} 818, Mazol^{®}, Sefsol^{®} 860 or Sefsol^{®} 870, Miglyol^{®} 812 being preferred. Miglyol^{®} 812 is a fractionated coconut oil comprising caprylic-capric acid triglycerides and having a relative molecular mass of about 520 Da. Fatty acid composition: C₆ maximum about 3%, C₈ about 50% to 65%, C₁₀ about 30% to 45%, C₁₂ maximum 5%; acid value about 0.1, saponification value about 330 to 345, maximum iodine value 1. Miglyol^{®} 812 is available from Condea. Neobee^{®} M 5 F is a fractionated caprylic-capric acid triglyceride available from coconut oil; maximum acid value 0.2, saponification value about 335 to 360, maximum iodine value 0.5, maximum water content 0.15%, D²⁰ 0.930-0.960, n_{D}²⁰ 1.448-1.451. Neobee^{®} M 5 F is available from Stepan Europe. A further example is Miglyol^{®} 829 containing additionally esters with succinic acid.

### 5) Glyceryl mono-C₁₆-C₁₈-fatty acid esters

These can be obtained esterifying glycerol with vegetable oil followed by molecular distillation. Monoglycerides suitable for use in a composition of the invention include both symmetric (β-monoglycerides) as well as asymmetric monoglycerides (α-monoglycerides. They also include both uniform glycerides (in which the fatty acid constituent is composed primarily of a single fatty acid) as well as mixed glycerides (in which the fatty acid constituent is composed of various fatty acids). The fatty acid constituent may include both saturated and unsaturated fatty acids having a chain length of from e. g. C₁₆ to C₁₈. Suitable examples include GMOrphic^{®} by Eastman, Rylo^{®} MG20 distilled monoglyceride by Danisco Ingredients or Monomuls^{®} 90-018 by Henkel. For example, GMOrphic^{®}-80 (glyceryl monooleate) exhibits the following characterising data: minimum monoglyceride content 94%, minimum C18:1 content 75%, maximum peroxide value 2.5, maximum C18:2 + C18:3 15%, maximum C16:0 + C18:0 + C20:0 10%, maximum water 2%, maximum acid value 3, iodine value 65-75, saponification value 155-165, maximum free glycerine 1%, hydroxy value 300-330.

### 6) Mixed mono-, di- and tri-glycerides

These include mixed mono-, di- and tri-glycerides, that are commercially available under the trade name Maisine^{®} from Gattefossé. They are transesterification products of corn oil and glycerol. Such products are comprised predominantly of linoleic and oleic acid mono-, di- and tri-glycerides together with minor amounts of palmitic and stearic acid mono-, di- and tri-glycerides (corn oil itself being comprised of ca. 56% by weight of linoleic acid, ca. 30% of oleic acid, ca. 10% of palmitic acid and ca. 3% of stearic acid constituents). Physical characteristics are: maximum free glycerol 10%, monoglycerides ca. 40%, diglycerides ca. 40%, triglycerides ca. 10%, free oleic acid content ca. 1%. Further physical characteristics are: maximum acid value 2, iodine value 85-105, saponification value 150-175, mineral acid content = 0. The fatty acid content for Maisine^{®} is typically: palmitic acid ca. 11%, stearic acid ca. 2.5%, oleic acid ca. 29%, linoleic acid ca. 56%, others ca. 1.5% (H. Fiedler, *loc*. *cit.,* volume 2, page 958).

Mixed mono-, di- and tri-glycerides preferably comprise mixtures of C₈ to C₁₀ or C₁₂ to C₂₀ fatty acid mono-, di- and tri-glycerides, especially mixed C₁₆ to C₁₈ fatty acid mono-, di- and tri-glycerides. The fatty acid components of the mixed mono-, di- and tri-glycerides may comprise both saturated and unsaturated fatty acid residues. Preferably, however, they are predominantly comprised of unsaturated fatty acid residues; in particular C₁₈ unsaturated fatty acid residues. Suitably the mixed mono-, di- and tri-glycerides comprise at least 60%, preferably at least 75%, more preferably at least 85%, by weight of C₁₈ unsaturated fatty acid (for example linolenic, linoleic and oleic acid) mono-, di- and tri-glycerides. Suitably the mixed mono-, di- and tri-glycerides comprise less than 20%, for example about 15%, or about 10%, by weight or less, of saturated fatty acid (for example palmitic and stearic acid) mono-, di- and tri-glycerides. Mixed mono-, di- and tri-glycerides are preferably predominantly comprised of mono- and di-glycerides; for example mono- and di-glycerides comprise at least 50%, more preferably at least 70%, by weight, based on the total lipophilic component. More preferably, the mono- and di-glycerides comprise at least 75%, for example about 80%, or about 85%, by weight of the lipophilic component. Preferably monoglycerides comprise from about 25% to about 50% by weight, based on the total lipophilic component. More preferably from about 30% to about 40%, for example from about 35 to about 40%, by weight of monoglycerides are present. Preferably diglycerides comprise from about 30% to about 60% by weight, based on the total lipophilic component. More preferably from about 40% to about 55%, for example from about 48% to about 50%, by weight of diglycerides are present. Triglycerides suitably comprise at least about 5%, but less than about 25%, by weight, based on the total lipophilic component. More preferably from about 7.5% to about 15%, for example from about 9 to about 12%, by weight of triglycerides are present. Mixed mono-, di- and tri-glycerides may be prepared by admixture of individual mono-, di- or tri-glycerides in appropriate relative proportion. Conveniently, however, they comprise trans-esterification products of vegetable oils, for example of almond oil, ground nut oil, olive oil, peach oil or palm oil, preferably of corn oil, sunflower oil or safflower oil, most preferably of corn oil, with glycerol. Such transesterification products can be generally obtained as described in GB-2,257,359 or WO-1994/09211. Preferably some of the glycerol is first removed to give a "substantially glycerol free batch" when soft gelatine capsules are to be made. Purified transesterification products of corn oil and glycerol provide particularly suitable mixed mono-, di- and tri-glycerides hereinafter referred to as "refined oil" and produced according to procedures described in GB-2,257,359 or WO-1994/09211.

### 7) Acetylated monoglycerides (C₁₈)

These include Myvacet^{®} 9-45.

### 8) Propylene glycol mono-fatty acid esters

The fatty acid constituent may include both saturated and unsaturated fatty acids having a chain length of from e. g. C₈ to C₁₂. Particularly suitable are propylene glycol mono-esters of caprylic and lauric acid, commercially available, e. g. under the trade names Sefsol^{®} 218, Capryol^{®}90 or Lauroglycol^{®}90, from e. g. Nikko Chemicals Co., Ltd. or Gattefossé or as Capmul^{®} PG-8 from Abitec Corporation. For example, Lauroglycol^{®}90 exhibits the following additional characterising data: maximum acid value 8, saponification value 200-220, maximum iodine value 5, maximum free propylene glycol content 5%, minimum monoester content 90%. Sefsol^{®} 218 exhibits the following additional characterising data: maximum acid value 5, hydroxy value 220-280 (H. Fiedler, *loc. cit.,* volume 2, page 906).

### 9) Propylene glycol mono- and di-fatty acid esters

These include Lauroglycol^{®} FCC and Capryol^{®} PGMC.

### 10) Propylene glycol diesters

Propylene glycol di-fatty acid esters, such as propylene glycol dicaprylate, are commercially available under the trade name Miglyol^{®} 840 from, e. g., Sasol (H. Fiedler, *loc*. *cit.,* volume 2, page 1008) or as Captex^{®} 200 from Abitec Corporation.

### 11) Propylene glycol monoacetate and propylene glycols diacetate

These are also suitable.

### 12) Transesterified ethoxylated vegetable oils

These include transesterified ethoxylated vegetable oils obtainable by reacting various natural vegetable oils, for example maize oil, kernel oil, almond oil, ground nut oil, olive oil, soybean oil, sunflower oil, safflower oil or palm oil, or mixtures thereof, with polyethylene glycols, that have an average relative molecular mass of from about 200 Da to about 800 Da, in the presence of an appropriate catalyst. These procedures are described in US-3,288,824. Transesterified ethoxylated corn oil is preferred.

Transesterified ethoxylated vegetable oils are commercially available under the trade name Labrafil^{®} (H. Fiedler, *loc. cit.,* volume 2, page 880). Examples are Labrafil^{®} M 2125 CS (from corn oil; having an acid value of less than about 2, a saponification value of 155 to 175, an HLB value of 3 to 4 and an iodine value of 90 to 110) and Labrafil^{®} M 1944 CS (from kernel oil; having an acid value of about 2, a saponification value of 145 to 175 and an iodine value of 60 to 90). Labrafil^{®} M 2130 CS (transesterification product of a C₁₂ to C₁₈ glyceride and polyethylene glycol; having a melting point of about 35°C to 40°C, an acid value of less than about 2, a saponification value of 185 to 200 and an iodine value of less than about 3) may also be used. Preferred is Labrafil^{®} M 2125 CS from, e. g., Gattefossé.

### 13) Sorbitan fatty acid esters

Such esters include, e. g., sorbitan mono-C₁₂-C₁₈-fatty acid esters or sorbitan tri-C₁₂-C₁₈-fatty acid esters, commercially available under the trade mark Span^{®} from, e. g., Uniqema. Especially preferred is e. g. Span^{®} 20 (sorbitan monolaurate) or Span^{®} 80 (sorbitan monooleate) (Fiedler, *loc. cit.,* volume 2, page 1430; "Handbook of Pharmaceutical Excipients", 2nd edition, editors A. Wade and P. J. Weller, 1994, joint publication of the American Pharmaceutical Association, Washington, USA, and The Pharmaceutical Press, London, England, page 473).

### 14) Esterified compounds of fatty acids and primary alcohols

These include esterified compounds of fatty acids having 8 to 20 carbon atoms and primary alcohols having 2 to 3 carbon atoms, for example isopropyl myristate, isopropyl palmitate, ethyl linoleate, ethyl oleate, ethyl myristate, etc., isopropyl myristate and isopropyl palmitate and particularly ethyl linoleate being preferable.

### 15) Glycerol triacetate or (1,2,3)-triacetin

This is obtainable by esterifying glycerin with acetic anhydride. Glycerol triacetate is commercially available as, e. g., Priacetin^{®} 1580 from Unichema International or Eastman Triacetin from Eastman, or from Courtaulds Chemicals Ltd.. Glycerol triacetate exhibits the following characterising data: relative molecular mass 218.03 Da, D^{20.3} 1.159-1.163, n_{D}²⁰ 1.430-1.434, maximum water content 0.2%, viscosity (25°C) 17.4 mPas, maximum acid value 0.1, saponification value about 766-774, minimum triacetin content 97% (H. Fiedler, *loc*. *cit.,* volume 2, page 1580; Handbook of Pharmaceutical Excipients, loc. cit., page 534).

### 16) Acetyl triethyl citrate

This is obtainable by esterification of citric acid and ethanol, followed by acetylation with acetic anhydride. Acetyl triethyl citrate is commercially available, e. g. under the trade name Citroflex^{®} A-2 from, e. g., Morflex Inc..

### 17) Tributylcitrate or acetyl tributyl citrate

These are also suitable.

### 18) Polyglycerol fatty acid esters

These have, for example, from 2 to 10, e. g. 6, glycerol units. The fatty acid constituent can include both saturated and unsaturated fatty acids having a chain length of e. g. C₈ to C₁₈. Particularly suitable is e. g. Plurol^{®} Oleique CC497 from Gattefossé, having a saponification value of 133-155 and a hydroxy value of 196-244. Further suitable polyglycerol fatty acid esters include diglyceryl monooleate (DGMO) and hexaglyn-5-O, commercially available, e. g., from Nikko Chemicals Co., Ltd..

### 19) PEG-fatty alcohol ethers

These include Brij^{®} 30, which is a polyoxyethylene(4)lauryl ether.

### 20) Fatty alcohols and fatty acids

Fatty acids/alcohols can be obtained by hydrolysing various animal or vegetable fats or oils, such as olive oil, followed by separation of the liquid acids/alcohols. The fatty acid/alcohol constituent can include both saturated and mono- or di-unsaturated fatty acids/alcohols having a chain length of from e. g. C₆ to C₂₀. Particularly suitable are, e. g., oleic acid, oleyl alcohol, linoleic acid, capric acid, caprylic acid, caproic acid, tetradecanol, dodecanol or decanol. Oleyl alcohol is commercially available under the trade mark HD-Eutanol^{®} V from, e. g., Henkel KGaA. Oleyl alcohol exhibits the following characterising data: maximum acid value 0.1, hydroxy value about 210, iodine value about 95, maximum saponification value 1, D²⁰ about 0.849, n_{D}²⁰ 1.462, relative molecular mass 268 Da, viscosity (20°C) about 35 mPas. Oleic acid exhibits the following characterising data: relative molecular mass 282.47 Da, D²⁰ 0.895, n_{D}²⁰ 1.45823, acid value 195-202, iodine value 85-95, viscosity (25°C) 26 mPas (H. Fiedler, *loc. cit.,* volume 2, page 1112; Handbook of Pharmaceutical Excipients, loc. cit., page 325).

### 21) Tocopherol and its derivatives (e. g. its acetate)

These include Coviox^{®} T-70, Copherol^{®} 1250, Copherol^{®} F-1300, Covitol^{®} 1360 and Covitol^{®} 1100.

### 22) Pharmaceutically acceptable oils

The lipophilic component can be, e. g., a pharmaceutically acceptable oil, preferably with an unsaturated component, such as a vegetable oil.

### 23) Alkylene polyol ethers or esters

These include C₃₋₅alkylene triol, in particular glycerol, ethers or esters. Suitable C₃₋₅alkylene triol ethers or esters include mixed ethers or esters, i. e. components including other ether or ester ingredients, for example transesterification products of C₃₋₅alkylene triol esters with other mono-, di- or poly-ols. Particularly suitable alkylene polyol ethers or esters are mixed C₃₋₅alkylene triol/poly-(C₂₋₄alkylene) glycol fatty acid esters, especially mixed glycerol/polyethylene- or polypropylene-glycol fatty acid esters. Especially suitable alkylene polyol ethers or esters include products obtainable by transesterification of glycerides, e. g. triglycerides, with poly-(C₂₋₄alkylene) glycols, e. g. polyethylene glycols, or, optionally, glycerol. Such transesterification products can generally be obtained by alcoholysis of glycerides, e. g. triglycerides, in the presence of a poly-(C₂₋₄alkylene) glycol, e. g. a polyethylene glycol, or, optionally, glycerol (to effect the transesterification from the glyceride to the polyalkylene glycol/glycerol component via polyalkylene glycolysis/ glycerolysis). In general, such a reaction is effected by reacting the indicated components (glyceride, polyalkylene glycol, optionally glycerol) at elevated temperature under an inert atmosphere with continuous agitation.

Preferred glycerides are fatty acid triglycerides, e. g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues. Preferred polyalkylene glycol materials are polyethylene glycols, in particular polyethylene glycols having a relative molecular mass of from ca. 500 to ca. 4'000 Da, e. g. from ca. 1'000 to ca. 2'000 Da.

Suitable alkylene polyol ethers or esters include mixtures of C₃₋₅alkylene triol esters, e. g. mono-, di- and tri-esters in variable relative amounts, and poly-(C₂₋₄alkylene) glycol mono- and di-esters, together with minor amounts of free C₃₋₅alkylene triol and free poly-(C₂₋₄alkylene) glycol. As hereinabove set forth, the preferred alkylene triol moiety is glyceryl. Preferred polyalkylene glycol moieties include polyethylene glycol, in particular having a relative molecular mass of from ca. 500 to ca. 4'000 Da, and preferred fatty acid moieties are, in particular saturated, C₁₀₋₂₂fatty acid ester residues.

Particularly suitable alkylene polyol ethers or esters include transesterification products of a natural or hydrogenated vegetable oil and a polyethylene glycol or, optionally, glycerol; or compositions comprising glyceryl mono-, di- and tri-C₁₀₋₂₂fatty acid esters and polyethylene glycol mono- and di-C₁₀₋₂₂fatty esters (optionally together with, e. g. minor amounts of, free glycerol and free polyethylene glycol).

Preferred vegetable oils, polyethylene glycols (or polyethylene glycol moieties) and fatty acid moieties in relation to the above definitions are as hereinbefore set forth.

Particularly suitable alkylene polyol ethers or esters for use in the present invention include those commercially available under the trade name Gelucire^{®} from, e. g., Gattefossé, in particular:
a) Gelucire^{®} 33/01, which melts at ca. 33-37°C and has a saponification value of ca. 230-255;
b) Gelucire^{®} 39/01, melting point ca. 37.5-41.5°C, saponification value ca. 225-245;
c) Gelucire^{®} 43/01, melting point ca. 42-46°C, saponification value ca. 220-240. These Gelucire^{®} products a) to c) all have a maximum acid value of 3.

The compositions of the invention may include mixtures of such alkylene polyol ethers or esters.

### 24) Hydrocarbons

These include e. g. squalene, available from, e. g., Nikko Chemicals Co., Ltd..

### 25) Ethylene glycol esters

These include Monthyle^{®} (ethylene glycol monostearate), available from, e. g., Gattefossé.

### 26) Pentaerythritol fatty acid esters and polyalkylene glycol ethers

These include, for example, pentaerythrite-dioleate, -distearate, -monolaurate, -monostearate and -polyglycol ether (Fiedler, *loc. cit.,* volume 2, pages 1158-1160, incorporated herein by reference).

Some of these lipophilic components, e. g. the ones of entries 1) to 3), 5), 6), 8), 9), 12), 13) and 19), display surfactant-like behaviour and may also be termed co-surfactants.

A lipophilic component (when present) preferably comprises in a composition of the invention an amount of from about 5% to about 85%, e. g. from about 10% to about 85%, preferably from about 10% to about 60%, more preferably from about 15% to about 60%, more preferably from about 15% to about 40%, e. g. from about 20% to about 40%, by weight of the total composition of the invention.

For those embodiments of the present invention, where a carrier medium comprises a hydrophilic component in addition to a lipophilic component and a surfactant, preferably the relative proportions of the lipophilic component, the hydrophilic component and the surfactant lie within the "microemulsion region" on a standard three way plot graph.

A composition of the invention may, optionally, include a hydrophilic component. A hydrophilic component comprises one or more hydrophilic substances. Suitable hydrophilic substances include:

### 1) Polyethylene glycol glyceryl C₆₋₁₀fatty acid esters

These fatty acid esters include mono-, di- and tri-fatty acid esters. The fatty acids can be saturated or unsaturated, having a chain length of, e. g., from C₈ to C₁₀. The polyethylene glycols may have, e. g., from 5 to 10, e. g. 7, CH₂-CH₂-O units. A particularly suitable fatty acid ester is polyethylene glycol (7) glyceryl monococoate, commercially available, e. g. under the trade name Cetiol^{®} HE, e. g. from Henkel KGaA. Cetiol^{®} HE has a D²⁰ of 1.05, an acid value of less than 5, a saponification value of about 95, a hydroxy value of about 180 and an iodine value of less than 5 (H. Fiedler, *loc. cit.,* volume 1, page 337). Also suitable is Lipestrol^{®} E-810.

### 2) N-alkylpyrrolidones

Particularly suitable is, e. g., N-methyl-2-pyrrolidone, e. g. commercially available under the trade name Pharmasolve^{®} from, e. g., International Specialty Products. N-methyl-2-pyrrolidone exhibits the following characterising data: relative molecular mass 99.1 Da, D²⁵ 1.027-1.028, minimum purity 99.85% (area % by GC; including methyl isomers) (H. Fiedler, *loc. cit.,* volume 2, page 1004).

### 3) Benzyl alcohol

This is commercially available from, e. g., Merck or may be obtained by distillation of benzyl chloride with potassium or sodium carbonate. Benzyl alcohol exhibits the following characterising data: relative molecular mass 108.14 Da, D²⁰ 1.043-1.049, n_{D}²⁰ 1.538-1.541 (H. Fiedler, *loc. cit.,* volume 1, page 238; Handbook of Pharmaceutical Excipients, *loc. cit*., page 35).

### 4) Triethyl citrate

It can be obtained esterifying citric acid and ethanol, and is commercially available, e. g. under the trade name Citroflex^{®} 2. In pharmaceutical grade it is available under the name TEC-PG/N from, e. g., Morflex Inc.. Particularly suitable is triethyl citrate, which exhibits the following characterising data: relative molecular mass 276.3, specific gravity 1.135-1.139, refractive index 1.439-1.441, viscosity (25°C) 35.2 mPas, assay (anhydrous basis) 99.0%-100.5%, maximum water 0.25% (H. Fiedler, *loc. cit.,* volume 1, page 371; Handbook of Pharmaceutical Excipients, *loc. cit.,* page 540).

### 5) Other hydrophilic substances

Other suitable hydrophilic substances include Transcutol^{®} (C₂H₅-[O-(CH₂)₂]₂-OH), glycofurol (also known as tetrahydrofurfuryl alcohol polyethylene glycol ether), 1,2-propylene glycol, dimethylisosorbide (Arlasolve^{®}), polyethylene glycols, triethylenglycol, ethyl acetate and ethyl lactate.

A hydrophilic component (when present) preferably comprises in a composition of the invention an amount of from about 5% to about 70%, e. g. from about 5% to about 60%, preferably from about 10% to about 60%, more preferably from about 10% to about 50%, more preferably from about 10% to about 40%, e. g. from about 10% to about 30%, by weight of the total composition of the invention.

A composition of the invention may comprise a lower alkanol, such as ethanol, as a hydrophilic co-component. The use of ethanol is not essential, however, ethanol has been found to be of particular advantage when a composition is to be manufactured in soft gelatine encapsulated form: The storage characteristics of such a composition are improved; in particular, the risk of active agent precipitation following encapsulation procedures is reduced. Thus, the shelf life stability may be extended by employing ethanol or some other such co-component as an additional ingredient of a composition of the invention. The ethanol (when present) preferably comprises in a composition of the invention an amount of up to about 60%, preferably an amount of from about 5% to about 30%, more preferably from about 5% to about 20%, by weight of the total composition of the invention.

A composition of the invention may preferably include a surfactant, which may reduce the interfacial tension and thereby provide thermodynamic stability. A surfactant comprises one or more surfactant substances. A surfactant substance may be a complex mixture containing side products or unreacted starting materials involved in the preparation of the surfactant substance, e. g., a surfactant substance made by polyoxyethylation may contain e. g. a side product, e. g. polyethylene glycol. A, or each, surfactant substance (when present) is preferably characterized by an HLB value (which is preferably the mean HLB value) of from about 8 to about 17, especially of from about 10 to about 17. Suitable surfactant substances include:

### 1) Reaction products of a natural or hydrogenated castor oil and ethylene oxide

The natural or hydrogenated castor oils may be reacted with ethylene oxide in a molar ratio of from about 1 : 35 to about 1 : 60, with optional removal of the polyethyleneglycol component from the reaction mixture. Various such surfactant substances are commercially available. Particularly suitable surfactant substances include polyethyleneglycol-hydrogenated castor oils available under the trade name Cremophor^{®}, e. g. Cremophor^{®} RH 40, which has a saponification value of about 50 to 60, an acid value of less than about 1, a water content (Fischer) of less than about 2%, an n_{D}⁶⁰ value of about 1.453-1.457 and an HLB value of about 14 to 16, and Cremophor^{®} RH 60, which has a saponification value of about 40 to 50, an acid value of less than about 1, an iodine value of less than about 1, a water content (Fischer) of about 4.5% to 5.5%, an n_{D}⁶⁰ value of about 1.453-1.457 and an HLB value of about 15 to 17. Especially preferred is Cremophor^{®} RH40. Other useful surfactant substances of this class are available under the trade names Nikkol^{®}, e. g. Nikkol^{®} HCO-40 or Nikkol^{®} HCO-60, Mapeg^{®}, e. g. Mapeg^{®} CO-40h, Incrocas^{®},e. g. Incrocas^{®} 40, Tagat^{®}, e. g. Tagat^{®} RH 40 (polyoxyethylene-glycerol-fatty acid esters) or Simulsol^{®} OL-50 (PEG-40 castor oil, which has a saponification value of about 55 to 65, a maximum acid value of 2, an iodine value of 25 to 35, a maximum water content of 8% and an HLB value of about 13; available from Seppic). Other suitable surfactant substances of this class include polyethyleneglycol-castor oils, such as Cremophor^{®} EL, which has a relative molecular mass (by steam osmometry) of about 1630 Da, a saponification value of about 65 to 70, an acid value of about 2, an iodine value of about 28 to 32 and an n_{D}²⁵ value of about 1.471. These surfactant substances are further described in H. Fiedler, *loc. cit..*

### 2) Polyoxyethylene-sorbitan-fatty acid esters

These include mono- and tri-lauryl, -palmityl, -stearyl and -oleyl esters of the type commercially available under the trade name Tween^{®} (H. Fiedler, *loc*. *cit.,* volume 2, pages 1615 ff) from Uniqema, e. g.:
Tween^{®} 20 [polyoxyethylene(20)sorbitanmonolaurate],
Tween^{®} 21 [polyoxyethylene(4)sorbitanmonolaurate],
Tween^{®} 40 [polyoxyethylene(20)sorbitanmonopalmitate],
Tween^{®} 60 [polyoxyethylene(20)sorbitanmonostearate],
Tween^{®} 65 [polyoxyethylene(20)sorbitantristearate],
Tween^{®} 80 [polyoxyethylene(20)sorbitanmonooleate],
Tween^{®} 81 [polyoxyethylene(5)sorbitanmonooleate] and
Tween^{®} 85 [polyoxyethylene(20)sorbitantrioleate].
Preferred in this class are Tween^{®} 20 and Tween^{®} 80.

### 3) Polyoxyethylene-fatty acid esters

These include polyoxyethylene-stearic acid esters of the type commercially available under the trade name Myrj^{®} from Uniqema (H. Fiedler, *loco cit.,* volume 2, page 1042), especially Myrj^{®} 52 having a D²⁵ of about 1.1, a melting point of about 40°C to 44°C, an HLB value of about 16.9, an acid value of about 0 to 1 and a saponification value of about 25 to 35.

### 4) Polyoxyethylene-polyoxypropylene co-polymers and block co-polymers, or poloxamers

These include the type commercially available under the trade names Pluronic^{®} and Emkalyx^{®} (H. Fiedler, *loc. cit.,* volume 2, page 1203). Especially preferred in this class is Pluronic^{®} F68 (poloxamer 188) from BASF, having a melting point of about 52°C and a relative molecular mass of about 6'800 to 8'975 Da. Also preferred is Synperonic^{®} PE L44 (poloxamer 124) from Uniqema.

### 5) Polyoxyethylene-mono-esters of saturated C₁₀ to C₂₂ hydroxyfatty acids

These include esters of, e. g., saturated C₁₈ hydroxyfatty acids, e. g. 12-hydroxystearic acid PEG esters, wherein the PEG component has a relative molecular mass of, e. g., about 600 to about 900 Da, e. g. of about 660 Da, e. g. Solutol^{®} HS 15 from BASF comprising about 70% by weight of polyethoxylated 12-hydroxystearate and about 30% by weight of the unesterified polyethylene glycol component [BASF technical leaflet MEF 151 E (1986)]. Solutol^{®} HS 15 has a hydrogenation value of 90 to 110, a saponification value of 53 to 63, a maximum acid number of 1 and a maximum water content of 0.5% by weight.

### 6) Polyoxyethylene- and polyoxypropylene-alkyl ethers

These include polyethylene glycol ethers of C₁₂ to C₁₈ alcohols, e. g. polyoxyl 2-, 10- or 20-cetyl ether, polyoxyl 23-lauryl ether, polyoxyl 20-oleyl ether or polyoxyl 2-, 10-, 20- or 100-stearyl ether, commercially available, e. g., under the trade mark Brij^{®} from Uniqema. Especially preferred are, e. g., Brij^{®} 35 (polyoxyl 23-lauryl ether) and Brij^{®} 98 (polyoxyl 20-oleyl ether) (H. Fiedler, *loc. cit.,* volume 1, page 259; Handbook of Pharmaceutical Excipients, *loc. cit.,* page 367). Similarly suitable are, e. g., polyoxyethylene-polyoxypropylene-alkyl ethers, e. g. polyoxyethylene-polyoxypropylene ethers of C₁₂ to C₁₈ alcohols, e. g. polyoxyethylene 20-polyoxypropylene 4-cetyl ether commercially available, e. g. under the trade mark Nikkol^{®} PBC 34 from Nikko Chemicals Co., Ltd. (H. Fiedler, *loc. cit.,* volume 2, page 1239). Polyoxypropylene fatty alcohol ethers, e. g. Acconon^{®} E, are also suitable.

### 7) Sodium alkyl-sulfates, -sulfonates and -aryl sulfonates

These include sodium lauryl sulfate (sodium dodecyl sulphate), commercially available, e. g., under the trade name Texapon^{®} K12 from Henkel KGaA.

### 8) Water soluble tocopheryl polyethylene glycol succinic acid esters (TPGS)

These include those with a polymerisation number of about 1'000, e. g. available from Eastman Fine Chemicals.

### 9) Polyglycerol fatty acid esters

These include, e. g., those with from about 10 to about 20, e. g. with about 10, glycerol units. The fatty acid constituent may include both saturated and unsaturated fatty acids having, e. g., a chain length of from C₈ to C₁₈. Particularly suitable are, e. g., decaglyceryl mono-laurate (decaglyn 1-L), -myristate (decaglyn 1-M) or -oleate (decaglyn 1-O), commercially available, e. g., from Nikko Chemicals Co., Ltd. (H. Fiedler, *loc*. *cit.,* volume 2, page 1228).

### 10) Alkylene polyol ethers or esters

These include C₃₋₅alkylene triol, in particular glycerol, ethers or esters. Suitable C₃₋₅alkylene triol ethers or esters include mixed ethers or esters, i. e. components including other ether or ester ingredients, for example transesterification products of C₃₋₅alkylene triol esters with other mono-, di- or poly-ols. Particularly suitable alkylene polyol ethers or esters are mixed C₃₋₅alkylene triol/poly-(C₂₋₄alkylene) glycol fatty acid esters, especially mixed glycerol/polyethylene- or polypropylene-glycol fatty acid esters. Especially suitable alkylene polyol ethers or esters include products obtainable by transesterification of glycerides, e. g. triglycerides, with poly-(C₂₋₄alkylene) glycols, e. g. polyethylene glycols, or, optionally, glycerol. Such transesterification products can generally be obtained by alcoholysis of glycerides, e. g. triglycerides, in the presence of a poly-(C₂₋₄alkylene) glycol, e. g. a polyethylene glycol, or, optionally, glycerol (to effect the transesterification from the glyceride to the polyalkylene glycol/glycerol component via polyalkylene glycolysis/ glycerolysis). In general, such a reaction is effected by reacting the indicated components (glyceride, polyalkylene glycol, optionally glycerol) at elevated temperature under an inert atmosphere with continuous agitation.

Preferred glycerides are fatty acid triglycerides, e. g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues. Preferred polyalkylene glycol materials are polyethylene glycols, in particular polyethylene glycols having a relative molecular mass of from ca. 500 to ca. 4'000 Da, e. g. from ca. 1'000 to ca. 2'000 Da.

Suitable alkylene polyol ethers or esters include mixtures of C₃₋₅alkylene triol esters, e. g. mono-, di- and tri-esters in variable relative amounts, and poly-(C₂₋₄alkylene) glycol mono- and di-esters, together with minor amounts of free C₃₋₅alkylene triol and free poly-(C₂₋₄alkylene) glycol. As hereinabove set forth, the preferred alkylene triol moiety is glyceryl. Preferred polyalkylene glycol moieties include polyethylene glycol, in particular having a relative molecular mass of from ca. 500 to ca. 4'000 Da, and preferred fatty acid moieties are, in particular saturated, C₁₀₋₂₂fatty acid ester residues.

Particularly suitable alkylene polyol ethers or esters include transesterification products of a natural or hydrogenated vegetable oil and a polyethylene glycol or, optionally, glycerol; or compositions comprising glyceryl mono-, di- and tri-C₁₀₋₂₂fatty acid esters and polyethylene glycol mono- and di-C₁₀₋₂₂fatty acid esters (optionally together with, e. g. minor amounts of, free glycerol and free polyethylene glycol).

Preferred vegetable oils, polyethylene glycols (or polyethylene glycol moieties) and fatty acid moieties in relation to the above definitions are as hereinbefore set forth.

Particularly suitable alkylene polyol ethers or esters for use in the present invention include those commercially available under the trade name Gelucire^{®} from, e. g., Gattefossé. The Gelucire^{®} products are inert, semi-solid, waxy materials with amphiphilic character, which may be identified, e. g., by their melting points and their HLB values. Mostly, the Gelucire^{®} products are saturated polyglycolised glycerides and fatty acid esters obtainable by polyglycolysis of natural or hydrogenated vegetable oils with polyethylene glycols. The Gelucire^{®} products are mixtures of mono-, di- and tri-glycerides and mono- and di-fatty acid esters of polyethylene glycols. Preferred are Gelucire^{®} 50/13 and, in particular, Gelucire^{®} 44/14. Gelucire^{®} 50/13 has a melting point of ca. 46-51 °C, a saponification value of ca. 67-81 and a maximum acid value of 2. Gelucire^{®} 44/14 has a melting point of ca. 42.5-47.5°C and an HLB value of 14 and exhibits the following additional characterising data: saponification value ca. 79-93, maximum acid value 2, maximum iodine value 2, hydroxy value 36-56, maximum peroxide value 6, maximum alkaline impurities 80, maximum water content 0.5% by weight, maximum free glycerol content 3% by weight. Gelucire^{®} 44/14 can be obtained by reacting hydrogenated palm kernels and/or hydrogenated palm oils with polyethylene glycol 1500. Gelucire^{®} 44/14 comprises approximately 20% by weight of mono-, di- and tri-glycerides and approximately 72% by weight of mono- and di-fatty acid esters of polyethylene glycol 1500, as well as approximately 8% by weight of free polyethylene glycol 1500. The fatty acids distribution for Gelucire^{®} 44/14 is as follows: 4-10 C₈, 3-9 C₁₀, 40-50 C₁₂, 14-24 C₁₄, 4-14 C₁₆, 5-15 C₁₈ (manufacturer information; H. Fiedler, *loc*. *cit.,* volume 1, page 676).

Alkylene polyol ethers or esters having a maximum iodine value of 2 are generally preferred.

A composition of the invention may include mixtures of such alkylene polyol ethers or esters.

### 11) Polyethylene glycol-glyceryl-fatty acid esters

The fatty acid esters may include mono-, di- and/or tri-fatty acid esters. The fatty acid constituent may include both saturated and unsaturated fatty acids having a chain length of, e. g., from C₁₂ to C₁₈. The polyethylene glycol may have, e. g., from 10 to 40, e. g. 15 or 30, CH₂-CH₂-O units. Particularly suitable is polyethylene glycol(15)-glyceryl monostearate, commercially available, e. g., under the trade name TGMS^{®}-15 from Nikko Chemicals Co., Ltd.. Other suitable esters include polyethylene glycol(30)glyceryl monooleate, commercially available, e. g., under the trade name Tagat^{®} O (H. Fiedler, *loc. cit.,* volume 2, pages 1502-1503), polyethylene glycol(20)-glyceryl monooleate (Tagat^{®} 02), polyethylene glycol(30)glyceryl monolaurate (Tagat^{®} L) and polyethylene glycol(20)glyceryl monolaurate (Tagat^{®} L2), all from Goldschmidt (H. Fiedler, *loc*. *cit.,* volume 2, pages 1502-1503). Also suitable is Tagat^{®} TO.

### 12) Sterols and derivatives thereof

These include cholesterols and derivatives thereof, in particular phytosterols, e. g. sitosterol, campesterol or stigmasterol, and ethylene oxide adducts thereof, for example soya sterols and derivatives thereof, e. g. polyethylene glycol sterols, e. g. polyethylene glycol phyto- or soya-sterols. The polyethylene glycols may have, e. g., from 10 to 40, e. g. 25 or 30, CH₂-CH₂-O units. Particularly suitable is polyethylene glycol(30)phytosterol, commercially available, e. g., under the trade name Nikkol^{®} BPS-30 from Nikko Chemicals Co., Ltd.. Also suitable is polyethylene glycol(25)soya sterol, commercially available, e. g., under the trade name Generol^{®} 122 E 25 from Henkel (H. Fiedler, *loc. cit.,* volume 1, page 680).

### 13) Transesterified polyoxyethylated caprylic-capric acid glycerides

These include, e. g., the product commercially available under the trade name Labrasol^{®} from Gattefossé. Labrasol^{®} has a maximum acid value of 1, a saponification value of 90-110 and a maximum iodine value of 1 (H. Fiedler, *loc. cit.,* volume 2, page 880).

### 14) Sugar fatty acid esters

These include those of C₁₂₋₁₈-fatty acids, e. g. sucrose monolaurate, e. g. Ryoto^{®} L-1695, commercially available from Mitsubishi-Kasei Food Corp..

### 15) PEG sterol ethers

These include, e. g., those having from 5 to 35, e. g. from 20 to 30, CH₂-CH₂-O units, e. g. Solulan^{®} C24, commercially available from Amerchol.

### 16) Dioctyl succinates

These include, e. g., dioctyl sodium sulfosuccinate, commercially available, e. g., under the trade mark Aerosol^{®} OT from American Cyanamid Co. (H. Fiedler, *loc. cit.,* volume 1, page 118), and di-(2-ethylhexyl) succinate (H. Fiedler, *loc. cit.,* volume 1, page 487).

### 17) Phospholipids

These include in particular lecithins (H. Fiedler, *loc. cit.,* volume 2, pages 910, 1184). Suitable are, in particular, soya bean lecithins.

### 18) Salts of fatty acids, fatty acid-sulfates and -sulfonates

These include, e. g., those of C₆-C₁₈-fatty acids, -fatty acid sulfates and -fatty acid sulfonates, commercially available, e. g., from Fluka.

### 19) Salts of acylated amino acids

These include those of C₆-C₁₈-acylated amino acids, e. g. sodium lauroyl sarcosinate, commercially available, e. g., from Fluka.

### 20) Medium- or long-chain alkyl ammonium salts

These include, e. g., C₆-C₁₈-alkyl ammonium salts, e. g. cetyl trimethyl ammonium bromide, commercially available, e. g., from E. Merck AG.

A surfactant (when present) preferably comprises in a composition of the invention an amount of from about 5% to about 90%, e. g. from about 15% to about 85%, preferably from about 20% to about 60%, more preferably from about 35% to about 55%, by weight of the total composition of the invention.

A composition of the invention may, optionally, include an antioxidant as an additive. An antioxidant comprises one or more antioxidant substances. Suitable antioxidant substances include ascorbyl palmitate, butyl hydroxy anisole (BHA), butyl hydroxy toluene (BHT) and tocopherols. Preferred is α-tocopherol (vitamin E).

A composition of the invention may, optionally, include, as a further additive, one or more antimicrobial agents, enzyme inhibitors, flavours, preservatives, stabilizers, sweeteners and/or further additive components.

An additive (an antioxidant and/or a further additive) (when present) preferably comprises in a composition of the invention an amount of from about 0.05% to about 10%, e. g. from about 0.05% to about 5%, by weight of the total composition of the invention. An antimicrobial agent, an antioxidant, an enzyme inhibitor, a preservative or a stabilizer (when present) typically comprises in a composition of the invention an amount of from about 0.05% to about 1% by weight of the total composition of the invention. A flavour or a sweetener (when present) typically comprises in a composition of the invention an amount of up to about 5%, e. g. up to about 2.5%, by weight of the total composition of the invention.

A composition of the invention may, optionally, include a component, that solidifies a liquid microemulsion preconcentrate, as a further additive component, e. g. a solid polyethylene glycol or a Gelucire^{®} product, preferably Gelucire^{®} 44/14 or Gelucire^{®} 50/13 described hereinbefore.

In a seventh aspect, the present invention relates to a process for the preparation of a spontaneously dispersible pharmaceutical composition comprising a cannabinoid receptor binding naphthalene derivative as an active agent and a carrier medium, which process comprises bringing the active agent, if present an antioxidant and/or a further additive, and the carrier medium comprising a lipophilic component, a surfactant, and optionally a hydrophilic component into intimate admixture.

The carrier medium can be prepared separately before bringing the active agent into intimate admixture with the carrier medium. Alternatively, the two or more of the components of the carrier medium can be mixed together with the active agent.

In an eighth aspect, the present invention relates to a process for the preparation of a microemulsion comprising a cannabinoid receptor binding naphthalene derivative as an active agent and a carrier medium, which process comprises
(i) bringing the active agent, if present an antioxidant and/or a further additive, and the carrier medium comprising a lipophilic component, a surfactant, and optionally a hydrophilic component into intimate admixture to form a spontaneously dispersible pharmaceutical composition and
(ii) diluting the spontaneously dispersible pharmaceutical composition in an aqueous medium, e. g. water, to form the microemulsion.

The relative proportions of the active agent, the lipophilic component, the surfactant and the hydrophilic component (when present) preferably lie within the "microemulsion region" on a standard three way plot graph. The compositions of the invention, e. g. those described in the examples hereinafter (which compositions are preferred), may show good stability characteristics as indicated by standard stability trials, for example having a shelf life stability of up to one, two or three years, or even longer. The compositions of the invention may, therefore, be of high stability and capable, on addition to an aqueous medium, e. g. water, of providing microemulsions, for example having a mean particle size of < 200 nm, e. g. <150 nm, e. g. < 100 nm.

When the composition of the invention is a microemulsion preconcentrate, it may be combined with water or an aqueous solvent medium to obtain a microemulsion. The microemulsion may be administered enterally, for example orally.

When the composition of the invention is a microemulsion preconcentrate, a unit dosage of the microemulsion preconcentrate is preferably used to fill orally administrable capsule shells. The capsule shells may be soft or hard capsule shells, for example made of gelatin. Each unit dosage will suitably contain from about 0.1 to about 100 mg, for example 0.1 mg, 1 mg, 2 mg, 5 mg, 10 mg, 15 mg, 20 mg, 25 mg, 50 mg, 75 mg or 100 mg, preferably from 1 to 100 mg, e. g. from between 10 to 100 mg, preferably from 2 to 75 mg, e. g. from 10 to 75 mg; for example 15, 20, 25, 50 or 75 mg, more preferably from 5 to 50 mg, e. g. 5 mg, 30 mg or 50 mg, preferably 5 mg or 30 mg, of active agent. Such unit dosage forms are suitable for administration, e. g., 1 to 5 times daily depending upon the particular purpose of therapy, the phase of therapy and the like. If desired, the compositions of the invention may be in drink solution form and may include water or any other aqueous system, e. g. fruit juice, milk, and the like, to provide e. g. colloidal systems, suitable for drinking, e. g. with a dilution of from about 1: 10 to about 1:100.

The compositions of the invention exhibit especially advantageous properties when administered orally; for example in terms of consistency and high level of bioavailability obtained in standard bioavailability trials.

Pharmacokinetic parameters, for example drug substance absorption, measured for example as blood levels, also may become surprisingly more predictable, and problems in administration with erratic absorption may be eliminated or reduced. Additionally, the compositions of the invention are effective with biosurfactants or tenside materials, for example bile salts, being present in the gastro-intestinal tract. That is, the compositions of the invention are fully dispersible in aqueous systems comprising such natural tensides and thus capable of providing emulsion or microemulsion systems and/or particulate systems in situ which are stable. The function of the compositions of the invention upon oral administration remains substantially independent of and/or unimpaired by the relative presence or absence of bile salts at any particular time or for any given individual. The compositions of the invention may also reduce variability in inter- and intra-subject, e. g. -patient, dose response.

The utility of the compositions of the invention may be observed in standard clinical tests in, for example, known indications of an active agent at dosages giving therapeutically effective active agent blood levels. Any increased bioavailability of the compositions of the invention may be observed in standard animal tests and in clinical trials.

The dose of the active agent in the compositions of the invention is of the same order as, or up to half, that used in known compositions containing the active agent. The compositions of the invention show activity at concentrations from about 0.1 mg to about 80 mg/day of active agent, preferably from about 0.1 mg to about 60 mg/day, e. g. most preferably from about 0.1 to about 40 mg/day of active agent in the treatment or prevention of chronic pain disease states.

The compositions of the invention are useful for the treatment or prevention of various chronic pain disease states. For these indications, the appropriate dosage will, of course, vary depending upon, for example, the particular composition of the invention employed, the host, the mode of administration, and the nature and severity of the condition being treated.

A typical dose for the active agent is from 0.1 to 50 mg/day for the treatment of chronic neuropathic pain.

Thus, in a ninth aspect, the present invention relates to a method of treating a subject, e. g. a patient, suffering from a disorder treatable with a cannabinoid receptor binding naphthalene derivative comprising administering a therapeutically effective amount of a composition of the invention to such subject in need of such treatment.

The invention is illustrated, but not limited, by the following Examples.

### Examples

In the compositions (microemulsion preconcentrates) of the Examples 1 to 18 the pharmaceutically active agent is naphthalen-1-yl-(4-pentyloxynaphthalen-1-yl)-methanone, i. e. compound A. The % values are % by weight of the total composition.

### Example 1

| Ingredient of composition | % | mg/unit |
|---|---|---|
| Compound A | 5.0 | 15.0 |
| Cremophor RH 40 | 42.5 | 127.5 |
| Corn oil glycerides | 17.0 | 51.0 |
| 1,2-propylene glycol | 25.5 | 76.5 |
| Ethanol | 10.0 | 30.0 |

### Example 2

| Ingredient of composition | % | mg/unit |
|---|---|---|
| Compound A | 5.0 | 15.0 |
| Tween 80 | 47.5 | 142.5 |
| Isopropyl myristate | 28.5 | 85.5 |
| Transcutol HP | 19.0 | 57.0 |

### Example 3

| Ingredient of composition | % | mg/unit |
|---|---|---|
| Compound A | 5.0 | 15.0 |
| Tween 80 | 42.5 | 127.5 |
| Isopropyl myristate | 25.5 | 76.5 |
| Transcutol HP | 17.0 | 51.0 |
| Ethanol | 10.0 | 30.0 |

### Example 4

| Ingredient of composition | % | mg/unit |
|---|---|---|
| Compound A | 5.0 | 15.0 |
| Cremophor RH 40 | 47.5 | 142.5 |
| Miglyol812 | 19.0 | 57.0 |
| Transcutol HP | 28.5 | 85.5 |

### Example 5

| Ingredient of composition | % | mg/unit |
|---|---|---|
| Compound A | 5.0 | 15.0 |
| Cremophor RH 40 | 42.5 | 127.5 |
| Miglyol812 | 17.0 | 51.0 |
| Transcutol HP | 25.5 | 76.5 |
| Ethanol | 10.0 | 30.0 |

### Example 6

| Ingredient of composition | % | mg/unit |
|---|---|---|
| Compound A | 5.0 | 15.0 |
| Tween 80 | 47.5 | 142.5 |
| Isopropyl myristate | 38.0 | 114.0 |
| Triethyl citrate | 9.5 | 27.0 |

### Example 7

| Ingredient of composition | % | mg/unit |
|---|---|---|
| Compound A | 5.0 | 15.0 |
| Tween 80 | 42.5 | 127.5 |
| Isopropyl myristate | 34.0 | 102.0 |
| Triethyl citrate | 8.5 | 25.5 |
| Ethanol | 10.0 | 30.0 |

### Example 8

| Ingredient of composition | % | mg/unit |
|---|---|---|
| Compound A | 5.0 | 15.0 |
| Vitamin E TPGS | 51.0 | 153.0 |
| Miglyol 812 | 17.0 | 53.0 |
| Transcutol HP | 17.0 | 51.0 |
| Ethanol | 10.0 | 30.0 |

### Example 9

| Ingredient of composition | % | mg/unit |
|---|---|---|
| Compound A | 10.0 | 15.0 |
| Tween 80 | 45.0 | 67.5 |
| Isopropyl myristate | 27.0 | 40.5 |
| Transcutol HP | 18.0 | 27.0 |

### Example 10

| Ingredient of composition | % | mg/unit |
|---|---|---|
| Compound A | 10.0 | 15.0 |
| Tween 80 | 40.0 | 60.0 |
| Isopropyl myristate | 16.0 | 24.0 |
| Transcutol HP | 24.0 | 36.0 |
| Ethanol | 10.0 | 15.0 |

### Example 11

| Ingredient of composition | % | mg/unit |
|---|---|---|
| Compound A | 10.0 | 15.0 |
| Cremophor RH 40 | 45.0 | 67.5 |
| Miglyol 812 | 18.0 | 27.0 |
| Transcutol HP | 27.0 | 40.5 |

### Example 12

| Ingredient of composition | % | mg/unit |
|---|---|---|
| Compound A | 10.0 | 15.0 |
| Cremophor RH 40 | 40.0 | 60.0 |
| Miglyol812 | 16.0 | 24.0 |
| Transcutol HP | 24.0 | 36.0 |
| Ethanol | 10.0 | 15.0 |

### Example 13

| Ingredient of composition | % | mg/unit |
|---|---|---|
| Compound A | 5.0 | 15.0 |
| Cremophor RH 40 | 51.0 | 153.0 |
| Corn oil glycerides | 25.5 | 76.5 |
| 1,2-propylene glycol | 8.5 | 25.5 |
| Ethanol | 10.0 | 30.0 |

### Example 14

| Ingredient of composition | % | mg/unit |
|---|---|---|
| Compound A | 2.50 | 15.0 |
| Cremophor RH 40 | 52.50 | 315.0 |
| Corn oil glycerides | 26.25 | 157.5 |
| 1,2-propylene glycol | 8.75 | 52.5 |
| Ethanol | 10.00 | 60.0 |

### Example 15

| Ingredient of composition | % | mg/unit |
|---|---|---|
| Compound A | 5.0 | 15.0 |
| Cremophor RH 40 | 42.4 | 127.2 |
| Corn oil glycerides | 17.0 | 51.0 |
| 1,2-propylene glycol | 25.5 | 76.5 |
| α-tocopherol | 0.10 | 0.3 |
| Ethanol | 10.0 | 30.0 |

### Example 16

| Ingredient of composition | % | mg/unit |
|---|---|---|
| Compound A | 5.0 | 15.0 |
| Cremophor RH 40 | 47.4 | 142.2 |
| Miglyol812 | 19.0 | 57.0 |
| Transcutol HP | 28.5 | 85.5 |
| α-tocopherol | 0.10 | 0.3 |

### Example 17

| Ingredient of composition | % | mg/unit |
|---|---|---|
| Compound A | 5.0 | 15.0 |
| Cremophor RH 40 | 42.4 | 127.2 |
| Miglyol812 | 17.0 | 51.0 |
| Transcutol HP | 25.5 | 76.5 |
| α-tocopherol | 0.10 | 0.3 |
| Ethanol | 10.0 | 30.0 |

### Example 18

| Ingredient of composition | % | mg/unit |
|---|---|---|
| Compound A | 2.50 | 15.0 |
| Cremophor RH 40 | 52.40 | 314.4 |
| Corn oil glycerides | 26.25 | 157.5 |
| 1,2-propylene glycol | 8.75 | 52.5 |
| α-tocopherol | 0.10 | 0.6 |
| Ethanol | 10.00 | 60.0 |

The compositions (microemulsion preconcentrates) of the Examples 1 to 18 are prepared by mixing the carrier components (solid carrier components are molten prior to using them) and dissolving the active agent in the carrier mixture with stirring.

## Claims

1. A spontaneously dispersible pharmaceutical composition comprising a cannabinoid receptor binding naphthalene derivative.

2. A spontaneously dispersible pharmaceutical composition comprising a cannabinoid receptor binding naphthalene derivative and a carrier medium comprising a lipophilic component, a surfactant, and optionally a hydrophilic component.

3. A pharmaceutical composition as claimed in claim 1 or claim 2, where the cannabinoid receptor binding naphthalene derivative is naphthalen-1-yl-(4-pentyloxynaphthalen-1-yl)-methanone.

4. A pharmaceutical composition comprising naphthalen-1-yl-(4-pentyloxynaphthalen-1-yl)-methanone as active agent and a carrier medium comprising a lipophilic component, a surfactant and optionally a hydrophilic component, said composition being in a form that is suitable for oral administration.

5. A composition as claimed in claim 2 or claim 4, wherein the lipophilic component comprises a fatty acid esterified with a primary alcohol, a medium chain fatty acid triglyceride or a refined glycerol-transesterified corn oil.

6. A composition as claimed in any one of claims 2, 4 and 5, wherein the hydrophilic component comprises propylene glycol, transcutol (diethylene glycol monoethyl ether) or triethyl citrate.

7. A composition as claimed in any one of claims 2, 4, 5 and 6, wherein the surfactant comprises a polyethyleneglycol-hydrogenated castor oil or a polyoxyethylene-sorbitan-fatty acid ester.

8. A spontaneously dispersible pharmaceutical composition comprising about 0.05 to about 20% by weight of naphthalen-1-yl-(4-pentyloxynaphthalen-1-yl)-methanone, about 5 to about 85 % by weight of a lipophilic component, about 5 to about 90 % by weight of a surfactant, and optionally about 5 to about 60 % by weight of a hydrophilic component, all weights based on the total composition.

9. A composition as claimed in any one of claims 1 to 8 in the form of a microemulsion preconcentrate.

10. A composition as claimed in any one of claims 1 to 8 in the form of a microemulsion.

11. A composition according to any preceding claim in unit dosage form.

12. A composition according to claim 11 in soft or hard gelatin encapsulated form.

13. A composition according to any preceding claim, which comprises an antioxidant and/or a further additive.

14. A composition as claimed in any one of claims 1 to 12, which comprises an antioxidant.

15. A method of treating a subject suffering from a disorder treatable with a cannabinoid receptor binding naphthalene derivative comprising administering a therapeutically effective amount of a pharmaceutical composition as claimed in any preceding claim to such subject in need of such treatment.

16. A process for the preparation of a spontaneously dispersible pharmaceutical composition comprising a cannabinoid receptor binding naphthalene derivative as an active agent and a carrier medium, which process comprises bringing the active agent, if present an antioxidant and/or a further additive, and the carrier medium comprising a lipophilic component, a surfactant, and optionally a hydrophilic component into intimate admixture.

17. A process for the preparation of a microemulsion comprising a cannabinoid receptor binding naphthalene derivative as an active agent and a carrier medium, which process comprises
(i) bringing the active agent, if present an antioxidant and/or a further additive, and the carrier medium comprising a lipophilic component, a surfactant, and optionally a hydrophilic component into intimate admixture to form a spontaneously dispersible pharmaceutical composition and
(ii) diluting the spontaneously dispersible pharmaceutical composition in an aqueous medium to form the microemulsion.
